# EUROPÄISCHE PATENTSCHRIFT

(11) **EP 4 164 473 B1**
(45) Veröffentlichungstag und Bekanntmachung des Hinweises auf die Patenterteilung: **08.05.2024**
(21) Anmeldenummer: 21729295.2
(22) Anmeldetag: 27.05.2021
(51) Int. Cl.: A61B 5/00, G01N 33/497, G01N 27/327

(54) **ELEKTROCHEMISCHE SENSORANORDNUNG, ATEMALKOHOLMESSGERÄT UND VERFAHREN ZUM BESTIMMEN EINER VITALITÄT VON ELEKTRODEN EINES ELEKTROCHEMISCHEN SENSORS**
ELECTROCHEMICAL SENSOR ARRANGEMENT, BREATHALYZER AND METHOD FOR DETERMINING A VITALITY OF ELECTRODES OF AN ELECTROCHEMICAL SENSOR
ENSEMBLE CAPTEUR ÉLECTROCHIMIQUE, ÉTHYLOMÈTRE ET PROCÉDÉ DE DÉTERMINATION D'UNE VITALITÉ D'ÉLECTRODES D'UN CAPTEUR ÉLECTROCHIMIQUE

(30) Priorität: 16.06.2020 DE 102020115804
(43) Veröffentlichungstag der Anmeldung: 19.04.2023
(73) Patentinhaber: Dräger Safety AG & Co. KGaA, 23560 Lübeck (DE)
(72) Erfinder: BAESLER, Malte, 23558 Lübeck (DE)
(86) Internationale Anmeldenummer: PCT/EP2021/064198
(87) Internationale Veröffentlichungsnummer: WO 2021/254760

(56) Entgegenhaltungen:
- DE-A1-102019 003 021
- DE-B3-102012 017 638
- US-A- 5 759 368
- US-A1- 2006 078 467
- US-A1- 2017 303 819

## Beschreibung

### Technisches Gebiet

Die vorliegende Erfindung bezieht sich auf eine elektrochemische Sensoranordnung für ein Atemalkoholmessgerät, auf ein entsprechendes Atemalkoholmessgerät sowie auf ein Verfahren zum Bestimmen einer Vitalität von Elektroden eines elektrochemischen Sensors, etwa zur Messung der Vitalität eines elektrochemischen Sensors.

### Hintergrund

Atemalkoholmessgeräte messen die Alkoholkonzentration im menschlichen Atem. Für mobile Messgeräte, aber teilweise auch für den evidentiellen Einsatz, werden elektrochemische Sensoren verwendet, deren Wirkprinzip die Brennstoffzelle ist. Die Empfindlichkeit des elektrochemischen Sensors kann durch Änderung der Umgebungsbedingungen variieren, insbesondere durch Alterung und Feuchteänderung oder Konzentrationsänderung des wässrigen Elektrolyten. Durch Austrocknung verlangsamen sich im Allgemeinen einige chemischen Vorgänge des Sensors und dieser wird langsamer. Durch Rückfeuchtung kann der Sensor wieder beschleunigt werden. Die Vorgänge Austrocknung und Rückfeuchtung sind dabei nicht vollständig reversibel, sodass der Sensor mit der Zeit degenerieren kann. Ebenso besitzt die Sensortemperatur einen Einfluss auf die Sensordynamik. Bei manchen Alkoholsensoren verdoppelt sich beispielsweise die Sensorgeschwindigkeit, wenn der Sensor um etwa 12°C heißer wird. Dabei ist eine Vitalität der Elektroden abhängig von einer Degenerierung des elektrochemischen Sensors aufgrund von Austrocknung. US 5 759 368 A offenbart ein Alkoholmessgerät mit einem Heizelement, welches ein Heizelement besitzt, um die chemische Reaktion des Sensors während der Messung zu beschleunigen und somit die Messzeit zu reduzieren.

Die Erkennung langsamer, degenerierter Sensoren erfolgt meist bei der Justierung, etwa über eine Auswertung der Sensorgeschwindigkeit mittels Begasung mit dem Zielgas. Hierbei wird beispielsweise die Auswertegeschwindigkeit bestimmt und diese, in manchen Fällen, temperaturkompensiert.

Der Nachteil der oben genannten Ansätze liegt darin, dass die Vitalitätserkennung nur zum Zeitpunkt der Justierung erfolgen kann. In der Zeit dazwischen, d.h. im Feldbetrieb, kann ein langsamer Sensor nicht erkannt werden. Wünschenswert aus Sicht des Kunden ist allerdings eine frühzeitige Warnung vor schwachen Sensoren.

### Zusammenfassung

Es besteht ein Bedarf nach einem verbesserten Konzept zum Bestimmen einer Vitalität eines elektrochemischen Sensors, das eine Bestimmung der Vitalität im Feldbetrieb ermöglicht. Diesem Bedarf wird durch die elektrochemische Sensoranordnung sowie das Verfahren der unabhängigen Ansprüche Rechnung getragen.

Die vorliegende Erfindung basiert auf der Erkenntnis, dass die Bestimmung der Vitalität der Elektroden eines elektrochemischen Sensors dadurch durchgeführt werden kann, dass selektiv eine der Elektroden des elektrochemischen Sensors erwärmt wird, sich dadurch eine Spannung zwischen den Elektroden bildet, und ein Stromfluss, der sich durch den Abbau dieser Spannung bildet, analysiert werden kann, um die Vitalität der Elektroden des elektrochemischen Sensors zu bestimmen. Eine solche selektive Erhitzung einer der Elektroden wird durch eine Wärmequelle durchgeführt, die als Teil der elektrochemischen Sensoranordnung vorgesehen ist. Somit ist die Erzeugung der entsprechenden Spannung unabhängig von einem Justageapparat auch im Feld möglich. Diese Eigenschaft kann ausgenutzt werden, um die entsprechende Vitalitätsbestimmung in kurzen Abständen selbst vorzunehmen. Die Bestimmung der Vitalität kann somit regelmäßig oder durch den Kunden manuell ausgelöst werden. Daraus ergibt sich, in zumindest einigen Ausführungsbeispielen, eine zuverlässige Vitalitätserkennung im Feld ohne Begasungstest. Dadurch kann der Kunde frühzeitig gewarnt werden, den Sensor auszutauschen (im Sinne einer vorausschauenden Wartung). Auf Basis der Vitalität können ferner Kompensationsparameter im Betrieb angepasst werden. Dadurch kann die Genauigkeit der Geräte verbessert werden. Durch die Anpassung der Parameter können die Justierintervalle verlängert werden, oder es kann bei einigen Geräten sogar ganz auf eine erneute Justierung verzichtet werden.

Die vorliegende Erfindung schafft eine elektrochemische Sensoranordnung für ein Atemalkoholmessgerät wie in Anspruch 1 definiert. Dabei ist die elektrochemische Sensoranordnung nicht auf Atemalkoholmessungen beschränkt. Im Gegenteil kann die elektrochemische Sensoranordnung auch in anderen Kontexten verwendet werden. Beispielsweise kann die elektrochemische Sensoranordnung eine elektrochemische Sensoranordnung für ein Gasmessgerät sein. Die elektrochemische Sensoranordnung umfasst einen elektrochemischen Sensor mit zumindest zwei Elektroden. Die elektrochemische Sensoranordnung umfasst ferner eine Wärmequelle. Die Wärmequelle ist derart angeordnet, dass sie, bei Aktivierung, selektiv eine der Elektroden des elektrochemischen Sensors erwärmt. Durch die selektive Erwärmung der einen Elektrode wird eine Spannung zwischen den beiden Elektroden erzeugt. Sind die Elektroden über einen Messwiderstand verbunden, so fließt ein Strom, welcher dann analysiert wird, um die Vitalität des jeweiligen elektrochemischen Sensors, beispielsweise im Feldbetrieb, zu bestimmen.

In zumindest einigen Ausführungsbeispiele ist die Wärmequelle eine Lichtquelle, etwa eine Licht-emittierende Diode (LED). Dadurch ist eine berührungslose Erwärmung der einen Elektrode möglich, wodurch die Erfindung auch mit bestehenden elektrochemischen Sensoren ohne bauliche Veränderungen des eigentlichen Sensors verwendet werden kann. Ein weiterer Vorteil der LED ist, dass diese einen hohen Wirkungsgrad besitzt und vornehmlich Licht emittiert. Dieses durchdringt ein Kunststoffgehäuse des Sensors nur mit geringer Dämpfung, und wird erst auf den dunklen Elektroden adsorbiert. Als Konsequenz wird eine der Elektroden selektiv erwärmt. Die Erwärmung erfolgt außerdem kontaktlos. Dadurch kann der Energieeintrag sehr schnell aus- und wieder eingeschaltet werden und der Sensor reagiert mit sehr steilen Strommesskurven.

Die selektive Erwärmung der einen Elektrode erzeugt eine Spannung zwischen den Elektroden. Diese Spannung resultiert wiederum in einem Stromfluss, der analysiert werden kann, um die Vitalität des elektrochemischen Sensors zu bestimmen.

Die elektrochemische Sensoranordnung umfasst ferner eine Steuervorrichtung. Die Steuervorrichtung ist ausgebildet, um die Vitalität der Elektroden basierend auf der selektiven Erwärmung der einen Elektrode zu bestimmen, basierend auf der erzeugten Spannung, und dem dadurch erzeugten Stromfluss.

Die Steuervorrichtung ist ausgebildet, um das nachfolgend vorgestellte Verfahren auszuführen. Dadurch wird die elektrochemische Sensoranordnung, oder das Atemalkoholmessgerät mit der elektrochemischen Sensoranordnung, in die Lage versetzt werden, die Vitalität der Elektroden im Feld zu bestimmen.

In manchen Ausführungsbeispielen umfasst die elektrochemische Sensoranordnung ferner eine grafische Ausgabeeinheit, um eine Information über eine Vitalität des Sensors auszugeben. Dabei ist die Information über die Vitalität der Elektroden basierend auf der selektiven Erwärmung der einen Elektrode bestimmt. Beispielsweise kann die Steuervorrichtung ausgebildet sein, um die Information über die Vitalität des Sensors über die grafische Ausgabeeinheit auszugeben. Dadurch kann einem Nutzer der elektrochemischen Sensoranordnung, etwa des Atemalkoholmessgeräts, die Information über die Vitalität bereitgestellt werden. Basierend auf dieser Information kann es dem Benutzer ermöglicht werden, zu entscheiden, wann eine Justierung oder eine Erneuerung des Sensors durchzuführen ist, oder ob der elektrochemische Sensor noch ausreichend genau ist.

Ausführungsbeispiele der vorliegenden Erfindung schaffen ferner ein Atemalkoholmessgerät umfassend die elektrochemische Sensoranordnung. Dadurch kann das Atemalkoholmessgerät in die Lage versetzt werden, die Vitalität des integrierten elektrochemischen Sensors zu testen.

Ausführungsbeispiele der vorliegenden Erfindung schaffen ferner ein Verfahren zum Bestimmen einer Vitalität von Elektroden eines elektrochemischen Sensors. Das Verfahren umfasst ein Aktivieren einer Wärmequelle über einen vordefinierten Zeitraum, um eine der Elektroden des Sensors selektiv zu erwärmen. Das Verfahren umfasst ferner ein Bestimmen eines Stromflusses zwischen den Elektroden des elektrochemischen Sensors. Der Stromfluss basiert auf einer Spannung, die von der einer selektiven Erwärmung der einen Elektrode durch die Wärmequelle hervorgerufen ist. In der Folge gibt es einen Strommesswiderstand zwischen den Elektroden. Das Verfahren umfasst ferner ein Bestimmen der Vitalität des Sensors basierend auf einer Signalform des Stromflusses. Ein solches Verfahren kann von Komponenten ausgeführt werden, die in dem Gerät integriert sein können, das den elektrochemischen Sensor umfasst, wodurch ein Test des elektrochemischen Sensors im Feld ermöglicht wird.

In manchen Ausführungsbeispielen umfasst das Verfahren ferner ein Bereitstellen einer Information über die Vitalität des Sensors über eine grafische Ausgabeeinheit. Basierend auf dieser Information kann es einem Benutzer des elektrochemischen Sensors ermöglicht werden, zu entscheiden, wann eine Justierung oder eine Erneuerung des Sensors durchzuführen ist, oder ob der elektrochemische Sensor noch ausreichend genau ist.

Beispielsweise kann die Information über die Vitalität des Sensors eine Information über eine geschätzte Restdauer einer Verwendbarkeit des elektrochemischen Sensors unter Berücksichtigung einer vorgegebenen minimalen Messgenauigkeit umfassen. Dies ermöglicht dem Benutzer eine Abschätzung darüber, wie lange er den entsprechenden Sensor im Gerät noch betreiben kann.

In verschiedenen Ausführungsbeispiele wird das Verfahren durch ein Gerät ausgeführt, das den elektrochemischen Sensor und die Wärmequelle umfasst. Dabei kann das Verfahren als Teil eines Selbst-Tests des Geräts ausgeführt werden. Dies ermöglicht eine regelmäßige Testung des elektrochemischen Sensors, ohne dafür eine separate Testvorrichtung zu benötigen.

In manchen Ausführungsbeispielen umfasst das Verfahren ferner ein Bestimmen eines Kompensationsparameters basierend auf der Vitalität des Sensors. Der Kompensationsparameter bildet ab, inwiefern sich die Vitalität des Sensors auf die Messungen des Sensors auswirkt. Das Verfahren kann ferner ein Durchführen von Messungen mit Hilfe des elektrochemischen Sensors unter Berücksichtigung des Kompensationsparameters umfassen. Beispielsweise kann für eine gegebene Vitalität des Sensors bekannt sein, inwiefern sich die gemessenen Werte von den eigentlichen Werten unterscheiden. Dieses Wissen kann genutzt werden, um basierend auf der bestimmten Vitalität des elektrochemischen Sensors den Kompensationsparameter zu bestimmen.

### Figurenkurzbeschreibung

Einige Beispiele von Vorrichtungen und/oder Verfahren werden nachfolgend bezugnehmend auf die beiliegenden Figuren näher erläutert. Es zeigen:
- Fig. 1: zeigt ein schematisches Diagramm eines Ausführungsbeispiels einer elektrochemischen Sensoranordnung sowie eines Atemalkoholmessgeräts mit einer solchen elektrochemischen Sensoranordnung;
- Fig. 2a: zeigt ein Flussdiagramm eines Ausführungsbeispiels eines Verfahrens zum Bestimmen einer Vitalität von Elektroden eines elektrochemischen Sensors;
- Fig. 2b: zeigt ein schematisches Blockdiagramm eines Ausführungsbeispiels einer Steuervorrichtung zum Bestimmen einer Vitalität von Elektroden eines elektrochemischen Sensors;
- Fig. 3a: zeigt Messwerte eines elektrochemischen Sensors in einem beispielhaften Versuchsaufbau;
- Fig. 3b: zeigt Messwerte eines gesunden und eines ausgetrockneten elektrochemischen Sensors in einem weiteren beispielhaften Versuchsaufbau;
- Fig. 3c: zeigt Ethanolkurven von einem vitalen und einem ausgetrockneten elektrochemischen Sensor in einem weiteren beispielhaften Versuchsaufbau; und
- Fig. 4: zeigt Messergebnisse eines Versuchsaufbaus, in dem eine Elektrode eines elektrochemischen Sensors alternativ von einer LED und von einem Lötkolben berührungslos erwärmt wurde.

### Beschreibung

Verschiedene Beispiele werden nun ausführlicher Bezug nehmend auf die beiliegenden Figuren beschrieben. In den Figuren können die Stärken von Linien, Schichten und/oder Bereichen zur Verdeutlichung übertrieben sein.

Weitere Beispiele können Modifikationen, Entsprechungen und Alternativen abdecken, die in den Rahmen der Offenbarung fallen. Gleiche oder ähnliche Bezugszeichen beziehen sich in der gesamten Beschreibung der Figuren auf gleiche oder ähnliche Elemente, die bei einem Vergleich miteinander identisch oder in modifizierter Form implementiert sein können, während sie die gleiche oder eine ähnliche Funktion bereitstellen.

Es versteht sich, dass, wenn ein Element als mit einem anderen Element "verbunden" oder "gekoppelt" bezeichnet wird, die Elemente direkt, oder über ein oder mehrere Zwischenelemente, verbunden oder gekoppelt sein können. Wenn zwei Elemente A und B unter Verwendung eines "oder" kombiniert werden, ist dies so zu verstehen, dass alle möglichen Kombinationen offenbart sind, d. h. nur A, nur B sowie A und B, sofern nicht explizit oder implizit anders definiert. Eine alternative Formulierung für die gleichen Kombinationen ist "zumindest eines von A und B" oder "A und/oder B". Das Gleiche gilt, mutatis mutandis, für Kombinationen von mehr als zwei Elementen.

Die vorliegende Offenbarung befasst sich mit einer elektrochemischen Sensoranordnung, etwa für ein Atemalkoholmessgerät, sowie mit einem Verfahren zur Bestimmung der Sensorvitalität von elektrochemischen Sensoren im Feld, im Speziellen von Alkoholsensoren, über eine thermische Anregung, etwa mittels Licht (z.B. von einer LED). Der Test kann jederzeit erfolgen und benötigt keinen Einsatz von Testgas.

Der elektrochemische Sensor arbeitet als Brennstoffzelle. Unter Einwirkung von beispielsweise Ethanol wird dieser elektrochemisch mit dem Luftsauerstoff verbrannt und dabei wird elektrische Energie freigesetzt. In einer beispielhaften Implementierung wird der Strom mittels eines 4.3 Ohm Shuntwiderstand und einem genauen 24 Bit Delta-Sigma-Wandler gemessen. Es werden dabei Ströme im Nano-Ampere-Bereich gemessen, d.h. es wird eine rauscharme Messelektronik benötigt.

Ist der elektrochemische Sensor thermisch im Gleichgewicht, so liegt keine Spannung an seinen Elektroden an und es fließt somit kein Strom. Der Messwert ist 0. Wird die Eine Elektrode des Sensors allerdings stärker erwärmt als die andere, liegt also ein Temperaturgradient im Sensor vor, so wird eine Thermospannung erzeugt, die sich in einem Stromfluss äußert. Der Nullpunkt des Sensors im Gerät wird dadurch verschoben. Dieser Vorgang ist im Allgemeinen unerwünscht, sodass für eine genaue Messung der Sensor thermisch im Gleichgewicht sein muss, damit sein Nullpunkt nicht driftet.

Werden zwei gleiche Elektroden in einem Elektrolyten unterschiedlich stark mit UV-Licht bestrahlt, so kann man eine Spannung zwischen beiden Elektroden messen. Dieser Effekt wurde im Jahr 1839 von Alexandre Edmond Becquerel entdeckt und ist nach ihm als Becquerel-Effekt beschrieben. Der elektrochemische Sensor und der Alkohol-Sensor im Speziellen, ist jedoch nichts weiter als so eine Zelle. Wird der elektrochemische Sensor mit UV-Licht bestrahlt, so wird eine Spannung auf den Elektroden erzeugt. Der Grund ist, dass durch die Bestrahlung Elektronen durch Photonenadsorption auf höhere Energieniveaus gehoben werden können. Diese führen bei einem elektrochemischen Sensor zu einer Potentialerhöhung.

Beide Effekte, d.h. der Stromfluss infolge einer Thermospannung sowie infolge einer photoelektrischen Spannung, können in dieser Erfindung ausgenutzt werden. Auf der einen Seite wird durch kurzfristige einseitige Erwärmung des Sensors eine Thermospannung erzeugt auf der anderen Seite wird durch Bestrahlung mittels UV-Licht eine photoelektrische Spannung erzeugt. Beide kurzfristigen Spannungserzeugungen führen zu einer Stromkurve, welche entsprechend ausgewertet werden können.

Wurde ein Sensor thermisch oder photoelektrisch aus dem Gleichgewicht gebracht, so ist ein schneller Sensor in der Lage, die Störung schnell auszugleichen. Ein langsamer Sensor benötigt hierfür mehr Zeit. Außerdem sieht die Dynamik eines schlechten Sensors anders aus.

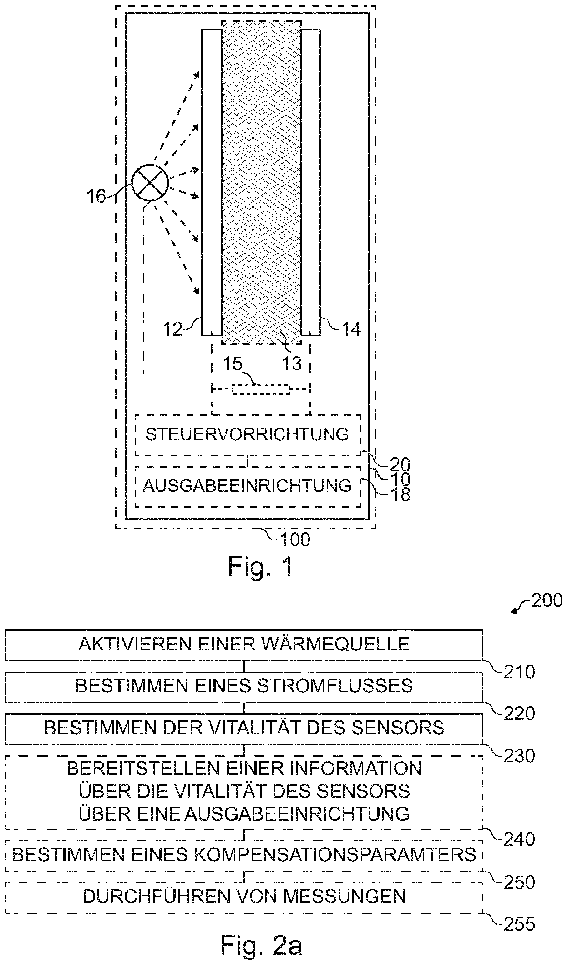

Fig. 1 zeigt ein schematisches Diagramm eines Ausführungsbeispiels einer elektrochemischen Sensoranordnung 10 sowie eines Atemalkoholmessgeräts 100 mit einer solchen elektrochemischen Sensoranordnung 10. Wie bereits zuvor erwähnt wurde ist das gleiche Prinzip auch auf elektrochemische Sensoranordnungen für Gassensoren anwendbar. Entsprechend zeigt Fig. 1 ferner einen Gassensor 100 mit einer solchen elektrochemischen Sensoranordnung 10. Die elektrochemische Sensoranordnung umfasst einen elektrochemischen Sensor mit zumindest zwei Elektroden 12; 14. Wie in Fig. 1 gezeigt ist umfasst der elektrochemische Sensor ferner ein Elektrolyt, das in dem Ausführungsbeispiel von Fig. 1 in einer Membran 13 vorgehalten wird, die zwischen der ersten Elektrode 12 und der zweiten Elektrode 14 angeordnet ist. Dabei kann der elektrochemische Sensor beispielsweise eine elektrochemische Brennstoffzelle sein, die dazu geeignet ist, eine Messung von Ethanol in menschlichem Atem durchzuführen. Alternativ kann der elektrochemische Sensor ein elektrochemischer Sensor zur Detektion von Gas sein. Dazu kann die elektrochemische Sensoranordnung ferner eine dritte Elektrode umfassen, etwa eine Referenzelektrode. Die elektrochemische Sensoranordnung 10 umfasst ferner eine Wärmequelle 16. Diese Wärmequelle ist derart angeordnet, dass sie, bei Aktivierung, selektiv genau eine der Elektroden des elektrochemischen Sensors erwärmt. Optional umfasst die elektrochemische Sensoranordnung ferner eine Steuervorrichtung 20, sowie eine Ausgabeeinrichtung 18. Dabei kann die Steuervorrichtung ausgebildet sein, um Messungen mit Hilfe des elektrochemischen Sensors durchzuführen. Dazu umfasst die elektrochemische Sensoranordnung ferner optional einen Messwiderstand 15, der zwischen den Anschlüssen der beiden Elektroden angeordnet ist. Die Ausgabeeinrichtung kann von der Steuervorrichtung angesteuert werden, um Messergebnisse und andere Informationen auszugeben. Ferner kann die Steuervorrichtung ausgebildet sein, um die Wärmequelle zu steuern. Daher kann die Steuervorrichtung beispielsweise mit den Elektroden, mit der Wärmequelle und mit der optionalen Ausgabeeinrichtung gekoppelt sein.

Der elektrochemische Sensor umfasst die zwei Elektroden 12; 14. Diese werden in einem elektrochemischen Sensor beispielsweise als Messelektrode und Gegenelektrode bezeichnet. Basierend auf einem Stromfluss zwischen diesen beiden Elektroden wird im Allgemeinen eine Messung mit Hilfe des elektrochemischen Sensors durchgeführt. Dabei wird, im Messbetrieb des elektrochemischen Sensors, ein Potential zwischen den Elektroden erzeugt. Ein Integral der daraus resultierende Stromkurve des gemessenen Stroms zwischen den Elektroden ist dabei, bei Verwendung in einem Atemalkoholmessgerät, proportional zum Atemalkohol (dem Ethanol in der Atemluft), und kann in einem nachfolgenden Verarbeitungsschritt verwendet werden, um den Atemalkohol zu bestimmen. Dabei entspricht das Integral des Stromflusses der Ladung, welches ein Maß für die Alkoholkonzentration ist. Zur Messung kann beispielsweise der Messwiderstand 15 verwendet werden.

Die elektrochemische Sensoranordnung umfasst ferner die Wärmequelle 16, die derart angeordnet ist, dass sie, bei Aktivierung, selektiv eine der Elektroden des elektrochemischen Sensors erwärmt. In anderen Worten ist die Wärmequelle dazu ausgebildet, selektiv die eine der beiden Elektroden zu erwärmen. Dabei bedeutet "selektiv", dass die Wärmequelle so angeordnet ist, dass sie eine der beiden Elektroden wesentlich mehr erwärmt als die andere, etwa so dass mindestens doppelt so viel Wärmeenergie von der einen Elektrode absorbiert wird wie von der anderen und/oder so dass ein Temperaturanstieg, etwa in Grad Celsius, in Folge der Erwärmung mindestens doppelt so hoch ist bei der einen Elektrode wie bei der anderen Elektrode. Im Idealfall wird, bei Aktivierung der Wärmequelle, ausschließlich die eine Elektrode erwärmt, dies wird, aufgrund einer Wärmeabstrahlung durch die eine Elektrode, nur in wenigen Implementierungen der Fall sein. Relevant ist dabei lediglich, dass eine der beiden Elektroden mehr erwärmt wird als die andere, so dass ein elektrisches Potential zwischen den Elektroden erzeugt wird. Durch die selektive Erwärmung der einen Elektrode wird also eine Spannung zwischen den Elektroden erzeugt. Dabei kommen sowohl verschiedene Arten von Wärmequellen als auch verschiedene Implementierungen eines Wärmetransfers in Betracht.

Beispielsweise kann die Wärmequelle eine Lichtquelle sein, etwa eine LED, oder aber auch eine Halogen-basierte Lichtquelle oder eine Glühlampe. Eine solche LED hat für die vorliegende Anwendung verschiedene Vorteile. Zum einen wird es dadurch ermöglicht, dass die eine Elektrode über einen vordefinierten Zeitraum erwärmt wird, ohne dass dabei die Wärmeausbreitung über einen Wärmeleiter berücksichtigt werden muss, da das Licht durch das Gehäuse dringt und vornehmlich direkt an den Elektroden adsorbiert wird. Um die schnelle Dynamik auswerten zu können, sollte die thermische Störung (Anregung) nur sehr kurzfristig anliegen. Eine thermische Heizung, die auf dem Prinzip der Wärmeleitung basiert, ist möglicherweise weniger geeignet, führt jedoch auch zu dem gewünschten Erfolg. Als besonders geeignet hat sich eine helle LED als Strahlungsquelle herausgestellt, insbesondere eine blaue LED. Diese sind mittlerweile für Leistungen bis zu 6W und einem Wirkungsgrad der Strahlung von über 70% erhältlich. Bei Experimenten wurde ferner festgestellt, dass die Energie einer UV-LED ausreichend ist, um eine Spannung, insbesondere eine photoelektrische Spannung, zu erzeugen. Ein Vorteil einer LED-Anregung ist, dass die thermische Energie zielgenau und sehr kurzfristig zum Sensor gebracht werden kann. Die Leistung kann praktisch ohne Latenz ein- und wieder ausgeschaltet werden. Zudem kann die Lichtquelle, etwa die LED, vollständig außerhalb des elektrochemischen Sensors angeordnet sein, wodurch das Konzept auch auf konventionelle elektrochemische Sensoren angewendet werden kann.

Beispielsweise kann die Wärmeübertragung oder die photoelektrische Beeinflussung der Elektrode drahtlos ausgeführt werden. Dabei wird unter drahtlos verstanden, dass die beeinflusste Elektrode nicht mit der Wärmequelle oder der Lichtquelle verdrahtet ist. Mit anderen Worten die Wärmeübertragung und/oder die Übertragung der Lichtenergie erfolgen berührungslos oder allgemein gesagt: die Energieübertragung erfolgt drahtlos und berührungslos. Dabei kann beispielsweise eine LED, ein thermischer Strahler, eine Glühbirne oder Induktion verwendet werden. Die Lichtquelle, etwa die LED, kann dabei so angeordnet sein, dass das bei Aktivierung der Lichtquelle emittierte Licht von der einen Elektrode absorbiert wird, von der anderen Elektrode jedoch nicht. In anderen Worten kann die Lichtquelle so ausgerichtet sein, dass das bei Aktivierung der Lichtquelle emittierte Licht auf die eine Elektrode gerichtet ist. Die andere Elektrode kann von dem emittierten Licht der Lichtquelle abgeschattet sein, etwa durch die eine Elektrode. Dazu kann die eine Elektrode 12 beispielsweise zwischen der Lichtquelle 16 und der anderen Elektrode 14 angeordnet sein.

Alternativ zu Lichtquellen können auch andere Arten von Wärmequellen verwendet werden, etwa Widerstandsheizer. Die Wärme kann in diesen Fällen mit Hilfe eines Wärmeleiters auf die eine Elektrode übertragen werden. In anderen Worten kann eine Wärmequelle in Kombination mit einem Wärmeleiter genutzt werden, um genau eine der Elektroden zu erhitzen.

In manchen Ausführungsbeispielen umfasst die elektrochemische Sensoranordnung, wie bereits zuvor ausgeführt wurde, eine Steuervorrichtung 20. Die Steuervorrichtung kann im Allgemeinen dazu ausgebildet sein, um Messungen mit Hilfe des elektrochemischen Sensors auszuführen. Die Steuervorrichtung kann ferner dazu ausgebildet sein, um die Vitalität der Elektroden basierend auf der selektiven Erwärmung der einen Elektrode zu bestimmen. Dazu kann die Steuervorrichtung einerseits dazu ausgebildet sein, um die Wärmequelle zu aktivieren, um die Spannung zwischen den Elektroden zu generieren. Andererseits kann die Steuervorrichtung dazu ausgebildet sein, um den auf der erzeugten Spannung basierenden Stromfluss zu messen, und basierend darauf die Vitalität des elektrochemischen Sensors zu bestimmen. Folglich kann die Steuervorrichtung ausgebildet sein, um die Vitalität der Elektroden ohne Nutzung eines Testgases zu bestimmen. Dazu kann die Steuervorrichtung beispielsweise ausgebildet sein, um das Verfahren von Fig. 2a auszuführen. Mehr Details zur Bestimmung der Vitalität werden daher im Zusammenhang mit dem Verfahren von Fig. 2a ausgeführt.

Im Allgemeinen kann die Steuervorrichtung 20, wie zuvor geschrieben wurde, auch verwendet werden, um Messungen mit Hilfe des elektrochemischen Sensors durchzuführen. Dazu kann die Steuervorrichtung 20 in einem Messbetrieb ausgebildet sein, um ein Potential zwischen den Elektroden des Sensors einzustellen. In einer elektrochemischen Brennstoffzelle ist ein Potential einer Messelektrode in Bezug auf die Bezugselektrode zu sehen, d.h. das Potential ist eine Potentialdifferenz zwischen dem Potential der Bezugselektrode und dem Potential der Messelektrode. Daher kann eine der Elektroden des elektrochemischen Sensors die Messelektrode sein, und die andere Elektrode kann die Bezugselektrode sein. Das Potential an der Messelektrode kann beispielsweise über eine sog. potentiostatische Regelschaltung eingestellt werden, eine Schaltung, bei der ein Stromfluss zwischen Messelektrode und Gegenelektrode erzeugt wird, um das Potential einzustellen. Die Steuervorrichtung kann beispielsweise eine potentiostatische Regelschaltung oder eine andere Regelschaltung umfassen, die dazu geeignet ist, das Potential einzustellen. Alternativ kann der elektrochemische Sensor als reine Brennstoffzelle verwendet werden, und der Stromfluss in der Brennstoffzelle bestimmt werden. Die Steuervorrichtung kann ferner ausgebildet sein, um eine Messung eines Stroms zwischen der Messelektrode und der Gegenelektrode während des Messbetriebs durchzuführen.

In zumindest einigen Ausführungsbeispielen umfasst die elektrochemische Sensoranordnung, oder das Gerät, das die elektrochemische Sensoranordnung umfasst, wie etwa das Atemalkoholmessgerät, eine grafische Ausgabeeinheit. Dabei sind mehrere Arten von grafischen Ausgabeeinheiten denkbar. Beispielsweise kann die grafische Ausgabeeinheit ein Bildschirm sein, etwa ein Flüssigkristallbildschirm oder ein Bildschirm, der auf einer organischen licht-emittierenden Dioden-Technologie (OLED) basiert. Alternativ kann die grafische Ausgabeeinheit auf einer Sieben-Segment-Anzeige oder auf ein oder mehreren Status-LEDs basieren. Die grafische Ausgabeeinheit kann dazu genutzt werden, um eine Information über die Vitalität des Sensors auszugeben. Beispielsweise kann die Steuervorrichtung ausgebildet sein, um die Information über die Vitalität des Sensors über die grafische Ausgabeeinheit auszugeben. Dabei ist die Information über die Vitalität der Elektroden basierend auf der selektiven Erwärmung der einen Elektrode bestimmt. Weitere Details dazu werden im Zusammenhang mit dem Verfahren von Fig. 2a vorgestellt.

In Ausführungsbeispielen kann die Steuervorrichtung 20 einem beliebigen Controller oder Prozessor oder einer programmierbaren Hardwarekomponente entsprechen. Beispielsweise kann die Steuervorrichtung 20 auch als Software realisiert sein, die für eine entsprechende Hardwarekomponente programmiert ist. Insofern kann die Steuervorrichtung 20 als programmierbare Hardware mit entsprechend angepasster Software implementiert sein. Dabei können beliebige Prozessoren, wie Digitale Signalprozessoren (DSPs) zum Einsatz kommen. Ausführungsbeispiele sind dabei nicht auf einen bestimmten Typ von Prozessor eingeschränkt. Es sind beliebige Prozessoren oder auch mehrere Prozessoren zur Implementierung der Steuervorrichtung 20 denkbar.

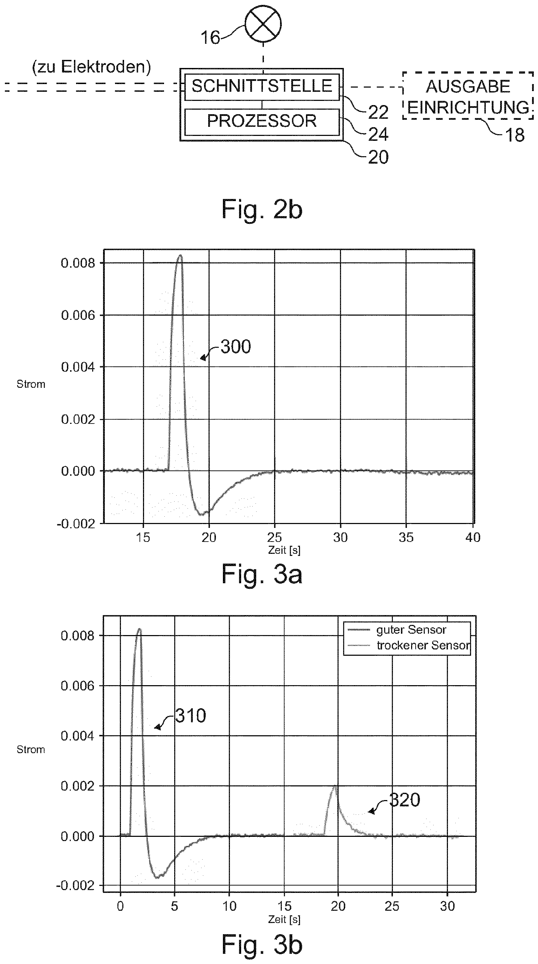

Mehr Details und Aspekte der elektrochemischen Sensoranordnung oder des Atemalkoholmessgeräts werden in Verbindung mit dem Konzept oder Beispielen genannt, die vorher oder nachher, z.B. Fig. 2, beschrieben werden. Die elektrochemische Sensoranordnung oder das Atemalkoholmessgerät kann ein oder mehrere zusätzliche optionale Merkmale umfassen, die ein oder mehreren Aspekten des vorgeschlagenen Konzepts oder der beschriebenen Beispiele entsprechen, wie sie vorher oder nachher beschrieben wurden.

Fig. 2a zeigt ein Flussdiagramm eines Ausführungsbeispiels eines Verfahrens 200 zum Bestimmen einer Vitalität von Elektroden eines elektrochemischen Sensors, etwa des elektrochemischen Sensors von Fig. 1 Das Verfahren umfasst ein Aktivieren 210 einer Wärmequelle, etwa der Wärmequelle 16 von Fig. 1, oder einer Strahlungsquelle über einen vordefinierten Zeitraum, um eine der Elektroden des Sensors, etwa die Elektrode 12 des elektrochemischen Sensors von Fig. 1, selektiv zu erwärmen. Das Verfahren umfasst ferner ein Bestimmen 220 eines Stromflusses zwischen den Elektroden des elektrochemischen Sensors. Der Stromfluss basiert auf einer Spannung, die von der einer selektiven Erwärmung der einen Elektrode durch die Wärmequelle hervorgerufen ist oder durch eine selektive Bestrahlung der einen Elektrode durch eine UV-Quelle. Das Verfahren umfasst ferner ein Bestimmen 230 der Vitalität des Sensors basierend auf einer Signalform des Stromflusses.

Fig. 2b zeigt ein schematisches Blockdiagramm eines Ausführungsbeispiels einer entsprechenden Steuervorrichtung 20 zum Bestimmen der Vitalität von Elektroden des elektrochemischen Sensors. Dabei kann die Steuervorrichtung 20 der Steuervorrichtung 20 entsprechend, wie sie im Zusammenhang mit Fig. 1 vorgestellt wurde. Die Steuervorrichtung kann beispielsweise eine Schnittstelle 22 und ein oder mehrere Prozessoren 24 umfassen, die mit der Schnittstelle gekoppelt sind. Dabei kann die Funktionalität der Steuervorrichtung von den ein oder mehreren Prozessoren bereitgestellt werden, wobei die Erfassung und Ausgabe von Signalen, etwa für und/oder von den Elektroden, der Wärmequelle oder der Ausgabeeinrichtung, über die Schnittstelle durchgeführt wird. Die Steuervorrichtung 20 ist ausgebildet zum Ausführen des Verfahrens der Fig. 2a. Insofern wird auch die Funktionalität der Steuervorrichtung im Folgenden mit Bezug auf das Verfahren erläutert. Generell kann das Verfahren beispielsweise von einem Gerät ausgeführt werden, das den elektrochemischen Sensor und die Wärmequelle umfasst, etwa von der Steuervorrichtung des Gerätes. Dabei kann das Gerät beispielsweise ein Atemalkoholmessgerät sein.

Das Verfahren umfasst das Aktivieren 210 der Wärmequelle über einen vordefinierten Zeitraum, um eine der Elektroden des Sensors selektiv zu erwärmen. Das Aktivieren der Wärmequelle kann beispielsweise ein Bereitstellen eines Steuersignals für die Wärmequelle, oder für eine Stromversorgung der Wärmequelle, umfassen, um die Wärmequelle zu aktivieren. Alternativ kann das Aktivieren der Wärmequelle ein Bereitstellen einer Stromversorgung für die Wärmequelle umfassen, etwa wenn die Wärmequelle direkt von der Steuervorrichtung mit ausreichend Strom versorgt werden kann. Dies kann beispielsweise der Fall sein, wenn die Wärmequelle eine LED ist, welche mit wenigen Watt Leistung betrieben werden kann. In manchen Ausführungsbeispielen kann der thermische Effekt genutzt werden, um eine Spannung zwischen den Elektroden zu erzeugen. Zusätzlich oder alternativ kann der photoelektrische Effekt genutzt werden. Daher kann die Wärmequelle so gesteuert werden, dass zum Erzeugen der Spannung zwischen den Elektroden eine der Elektroden über einen vordefinierten Zeitraum von der Wärmequelle thermisch oder auch photoelektrisch angeregt wird, etwa durch Emittieren von Licht auf die eine Elektrode.

Das Verfahren umfasst ferner das Bestimmen 220 des Stromflusses zwischen den Elektroden des elektrochemischen Sensors. Dabei basiert der Stromfluss, wie schon im Zusammenhang mit Fig. 1 ausgeführt wurde, auf der Spannung, die von der einer selektiven Erwärmung der einen Elektrode durch die Wärmequelle hervorgerufen ist. Das Bestimmen des Stromflusses kann dabei ähnlich wie die Messung des Stroms zwischen Messelektrode und Gegenelektrode im Messbetrieb eines elektrochemischen Sensoranordnung, die den elektrochemischen Sensor umfasst, durchgeführt werden. Der gemessene Strom kann dabei über eine Mehrzahl von Abtastpunkten (Sampeln) protokolliert werden und für eine nachfolgende Analyse in einem Speicher, etwa der Steuervorrichtung, vorgehalten werden.

Das Verfahren umfasst ferner das Bestimmen 230 der Vitalität des Sensors basierend auf der Signalform des Stromflusses. Die Signalform des Stromflusses kann dabei beispielsweise auf der Mehrzahl von protokollierten Abtastpunkten bestimmt werden. Folglich kann das Bestimmen der Vitalität des Sensors ein Bestimmen der Signalform basierend auf der Mehrzahl von protokollierten Abtastpunkten des gemessenen Stroms umfassen. Steht die Signalform zur Verfügung, so können ein oder mehrere Parameter der Signalform analysiert werden, um die Vitalität des elektrochemischen Sensors zu bestimmen.

Ein Parameter bezieht sich auf die Dauer, die benötigt wird, um die erzeugte Spannung wieder abzubauen. Folglich kann die Vitalität des Sensors basierend auf einer Zeitspanne zwischen einem ersten Zeitpunkt, an dem die Spannung erzeugt wurde und einem zweiten Zeitpunkt, an dem der Stromfluss nach dem Erzeugen der Spannung unter einen Schwellenwert gefallen ist, bestimmt werden. Dabei kann die Zeitspanne indikativ für die Vitalität der Elektroden sein. Je kürzer die Zeitspanne ist, desto "schneller" ist der elektrochemische Sensor, und desto höher ist die Vitalität des Sensors.

Zusätzlich oder alternativ kann die Form einer Signalspitze der Signalform genutzt werden, um die Vitalität des Sensors zu bestimmen. Beispielsweise kann die Vitalität des Sensors basierend auf einer Höhe oder Steilheit der Signalspitze in der Signalform bestimmt werden. Je höher die Signalspitze ist, oder je steiler die Signalspitze ist, desto höher ist die Vitalität des Sensors. Alternativ oder zusätzlich kann ein Anteil des Signalform, der der Signalspitze folgt, berücksichtigt werden. Elektrochemische Sensoren mit einer höhen Vitalität sind beispielsweise dadurch erkennbar, dass der Signalspitze ein Unterschwinger folgt, der bei ausgetrockneten elektrochemischen Sensoren nicht zu sehen ist. Folglich kann die Vitalität der Elektroden basierend auf einem Unterschwingen in der Signalform nach einer Signalspitze in der Signalform bestimmt werden.

In verschieden Ausführungsbeispielen umfasst das Verfahren ferner ein Bereitstellen 240 einer Information über die Vitalität des Sensors über eine grafische Ausgabeeinheit. Dabei wurden verschiedene Implementierungen der grafischen Ausgabeeinheit bereits im Zusammenhang mit Fig. 1 vorgestellt. Insbesondere kann die grafische Ausgabeeinheit ein Bildschirm sein, eine Sieben-Segment-Anzeige, oder eine grafische Ausgabeeinheit, die auf ein oder mehreren LEDs basiert. Die Information über die Vitalität des Sensors kann dabei die Vitalität des Sensors verschiedentlich repräsentieren. In einer ersten Implementierung kann die Information über die Vitalität des Sensors anzeigen, ob der elektrochemische Sensor ausreichend vital ist (und somit auch ausreichend genau oder schnell genug, um im Feldbetrieb genutzt zu werden). In diesem Fall kann die Information über die Vitalität des Sensors beispielsweise über eine einzelne LED dargestellt werden (wobei etwa ein grünes Leuchten der LED bedeutet, dass der elektrochemische Sensor ausreichend vital ist, und ein rotes Leuchten der LED bedeutet, dass der elektrochemische Sensor nicht mehr ausreichend vital ist). In einer weiteren Implementierung können mehr als zwei Zustände unterschieden werden. So kann beispielsweise die Information über die Vitalität eine von drei Zuständen anzeigen - gut, mittel oder schlecht, oder "Einwandfrei", "noch ausreichend vital (aber nicht mehr einwandfrei)", "nicht mehr ausreichend vital". In diesem Fall kann die entsprechende Information über die Vitalität auch über eine LED ausgegeben werden (etwa "grün", "gelb", "rot"), oder über einen Bildschirm des Geräts. Über einen Bildschirm des Geräts kann die Vitalität des Sensors auch in einer Prozentanzeige oder eine Balkendarstellung ausgegeben werden. Beispielsweise kann die Ausgabe bereitgestellt werden, falls die Vitalität der Elektroden einen Schwellenwert verletzt, etwa falls der Zustand "noch ausreichend vital" oder "nicht mehr ausreichend vital" ist, oder falls ein berechneter Prozentwert den Schwellenwert verletzt. In anderen Fällen kann die Ausgabe unterbleiben. In verschiedenen Implementierungen kann die Vitalitätserkennung im Feld durchgeführt werden, wobei im Sinne einer vorbeugenden Instandhaltung eine rechtzeitige Warnung des Kunden bei schwachem Sensor ausgegeben werden kann. Beispielsweise kann ein regelmäßiger Vitalitätscheck durchgeführt werden, z.B. beim Start des Geräts.

In manchen Ausführungsbeispielen kann die Information über die Vitalität auch, abgesehen von der Information über die aktuelle Vitalität des Sensors, eine Information über eine Prädikation eines Verlaufs der Vitalität umfassen. Eine solche Projektion kann nützlich sein, da die Vitalität des Sensors einen Einfluss hat auf die Genauigkeit des Sensors - verändert sich nur die Vitalität des Sensors, so verändert sich auch die geschätzte Genauigkeit. Basierend auf der Projektion lässt sich so feststellen, wie lange der Sensor noch ausreichend genau ist, unter Berücksichtigung einer vorgegebenen minimalen Messgenauigkeit. Beispielsweise kann die Signalform verwendet werden, um nicht nur die aktuelle Vitalität des Sensors zu bestimmen, sondern auch um zu bestimmen, wie lange der Sensor noch ausreichend vital ist. Dazu kann das Bestimmen 230 der Vitalität des Sensors ein Projizieren der Vitalität des Sensors umfassen, etwa basierend auf einer Veränderung der Signalform, und der entsprechend bestimmten Vitalität des Sensors, über mehrere Messungen hinweg, wobei die mehreren Messungen beispielsweise über mehrere Tage, Wochen oder Monate hinweg stattgefunden haben. Beispielsweise kann das Projizieren der Vitalität des Sensors das Durchführen einer Zeitserienprojektion basierend auf der Veränderung der Vitalität des Sensors über mehrere Messungen hinweg umfassen. Dazu kann beispielsweise ein Fitting-Algorithmus verwendet werden. Basierend auf der Projektion kann bestimmt werden, wie lange der Sensor, unter Berücksichtigung einer vorgegebenen minimalen Messgenauigkeit umfasst, voraussichtlich noch verwendbar ist. Beispielsweise kann eine Nachschlagetabelle verwendet werden, um von der Projektion der Vitalität des Sensors auf eine Projektion der geschätzten Genauigkeit des elektrochemischen Sensors zu schließen. Diese Schätzung kann anschließend mit einer vorgegebenen minimalen Messgenauigkeit verglichen werden. Folglich kann die Information über die Vitalität des Sensors eine Information über eine geschätzte Restdauer einer Verwendbarkeit des elektrochemischen Sensors unter Berücksichtigung einer vorgegebenen minimalen Messgenauigkeit umfassen. Dabei kann die Information über die geschätzte Restdauer der Verwendbarkeit beispielsweise eine Granularität von Monaten aufweisen, zudem kann die Schätzung konservativ ausgelegt werden. Beispielsweise kann die Information über die geschätzte Restdauer die Verwendbarkeit anzeigen, dass der elektrochemische Sensor noch zumindest eine angezeigte Anzahl von Monaten verwendbar ist, bevor der elektrochemische Sensor gewartet oder ausgetauscht werden muss. Hier kann ebenfalls die Ausgabe bereitgestellt werden, falls die geschätzte Restdauer unter einem Schwellenwert liegt.

In manchen Ausführungsbeispielen kann die ermittelte Vitalität des Sensors auch verwendet werden, um die Ergebnisse der Messungen, die mit Hilfe des Sensors durchgeführt werden, anzupassen und damit die Genauigkeit zu erhöhen. Die Genauigkeit des Sensors, wie bereits zuvor ausgeführt, von der Vitalität des Sensors abhängig. Dabei gibt es in vielen Fällen eine Abhängigkeit zwischen der Vitalität des Sensors und einer Abweichung des Messergebnisses. Diese Abweichung kann beispielsweise bauartbedingt sein - ist die Vitalität des Sensors bekannt, so kann daraus ein Kompensationsparameter berechnet werden, um diese Abweichung zu kompensieren. Folglich kann das Verfahren ein Bestimmen 250 eines Kompensationsparameters basierend auf der Vitalität des Sensors umfassen. Der Kompensationsparameter kann abbilden, inwiefern sich die Vitalität des Sensors auf die Messungen des Sensors auswirkt. Es kann somit eine Anpassung des Kompensationsparameters zwecks Verbesserung der Genauigkeit durchgeführt werden. Dadurch können längere Justierintervalle durch die verbesserte Genauigkeit erzielt werden. Beispielsweise kann der Kompensationsparameter die zuvor genannte Abweichung abbilden. So lässt sich der Kompensationsparameter beispielsweise basierend auf der bestimmten Vitalität berechnen, etwa basierend auf einer mathematischen Funktion oder basierend auf einer weiteren Nachschlagetabelle. Das Verfahren kann ferner ein Durchführen 255 von Messungen mit Hilfe des elektrochemischen Sensors unter Berücksichtigung des Kompensationsparameters umfassen. Dabei können die Messergebnisse der Messung auf dem Kompensationsparameter basieren. Durch die Nutzung des vorgeschlagenen Konzepts können die Vitalitätsparameter im Feld bestimmt und die Kompensationsparameter, welche für die Auswertung des Messergebnisses einer Alkoholmessung benötigt werden, angepasst werden. Dadurch steigt die Genauigkeit der Messung an. Diese verbesserte Genauigkeit kann zudem genutzt werden, um die geschätzte Restdauer der Verwendbarkeit des elektrochemischen Sensors zu berechnen. Somit kann auch ein Justierintervall verlängert werden. In manchen Implementierungen kann auch eine Bestimmung der Genauigkeit im Feld ohne Alkohol durchgeführt werden, oder die Justierung kann im Feld nachgezogen werden, etwa basierend auf dem Kompensationsparameter.

Die Bestimmung der Vitalität kann durch verschiedene Anlässe ausgelöst werden. Beispielsweise kann die Bestimmung der Vitalität zu regelmäßigen Zeitpunkten, etwa bei einem Starten des Geräts, durchgeführt werden. Dabei kann das Bestimmen der Vitalität beispielsweise ein Teil eines Selbst-Tests des Geräts sein. In anderen Worten kann das Verfahren durch ein Gerät ausgeführt werden, das den elektrochemischen Sensor und die Wärmequelle und entsprechend die Steuervorrichtung umfasst. Das Verfahren, etwa die Bestimmung der Vitalität des Sensors, kann als Teil eines Selbst-Tests des Geräts ausgeführt werden. Der Selbst-Test des Geräts kann beispielsweise bei einem Start des Geräts, oder auf Anforderung eines Benutzers des Geräts ausgeführt werden.

In manchen Ausführungsbeispielen kann das Bestimmen der Vitalität ferner genutzt werden, um festzustellen, ob der Sensor vorschriftsmäßig angeschlossen und funktionstüchtig ist. Dies kann beispielsweise mit einem Funktionstest geschehen, bei dem überprüft wird, ob ein Potential zwischen den Elektroden aufgebaut werden kann, und ob ein Stromfluss zwischen den Elektroden gemessen werden kann. Durch das vorgeschlagene Konzept kann zudem eine physische Sensorerkennung, z.B. durch die Auswertung der Sensorantwort durch einen Spannungspuls, mit ersetzt werden. Dabei ist die Erfindung nicht nur auf Alkoholsensoren beschränkt, sondern sie kann auch auf andere elektrochemische Sensoren angewendet werden. Das Verfahren kann ein Durchführen eines Funktionstests des elektrochemischen Sensors umfassen, wobei der Funktionstest auf der Bestimmung der Vitalität der Elektroden basiert. Wird bei der Bestimmung der Vitalität der Elektroden festgestellt, dass ein Stromfluss zwischen den Elektroden gemessen wird, so kann der Funktionstest als bestanden gelten.

Die Schnittstelle 22 kann beispielsweise einem oder mehreren Eingängen und/oder einem oder mehreren Ausgängen zum Empfangen und/oder Übertragen von Informationen entsprechen, etwa in digitalen Bitwerten, basierend auf einem Code, innerhalb eines Moduls, zwischen Modulen, oder zwischen Modulen verschiedener Entitäten.

In Ausführungsbeispielen können die ein oder mehreren Prozessoren 24 einem beliebigen Controller oder Prozessor oder einer programmierbaren Hardwarekomponente entsprechen. Beispielsweise kann die Funktionalität der ein oder mehreren Prozessoren 24 auch als Software realisiert sein, die für eine entsprechende Hardwarekomponente programmiert ist. Dabei können beliebige Prozessoren, wie Digitale Signalprozessoren (DSPs) zum Einsatz kommen. Ausführungsbeispiele sind dabei nicht auf einen bestimmten Typ von Prozessor eingeschränkt.

Mehr Details und Aspekte des Verfahrens und der Steuervorrichtung werden in Verbindung mit dem Konzept oder Beispielen genannt, die vorher, z.B. in Fig. 1, beschrieben wurden. Das Verfahren und die Steuervorrichtung können ein oder mehrere zusätzliche optionale Merkmale umfassen, die ein oder mehreren Aspekten des vorgeschlagenen Konzepts oder der beschriebenen Beispiele entsprechen, wie sie vorher oder nachher beschrieben wurden.

Es wurden Labormessungen zum Nachweis der Methode durchgeführt. Hierfür wurde eine Vorrichtung gebaut, um die Rohwerte des elektrochemischen Sensors aufzuzeichnen und eine blaue LED anzusteuern. Die LED wurde über den EC-Sensor platziert und für genau 1000 ms mit verminderter Leistung, z.B. 500 mA, stromgeregelt, betrieben. Diese Bestrahlung führt infolge der Thermospannung zu einer Änderung des Messwerts es elektrochemischen Sensors.

In einem ersten Test wurde ein gesunder Sensor vermessen. Fig. 3a zeigt Messwerte 300 eines gesunden elektrochemischen Sensors in einem beispielhaften Versuchsaufbau. Der Sensorreagiert deutlich auf die Bestrahlung. Nach weniger als einer Sekunde hat die abklingende Flanke bereits die Nulllinie durchschritten. Außerdem ist ein charakteristisches Unterschwingen zu erkennen.

Für einen weiteren Versuch wurde ein elektrochemischer Sensor künstlich getrocknet. Im Anschluss wurden Messwerte 310 eines gesunden (vitalen) elektrochemischen Sensors und Messwerte 320 eines ausgetrockneten elektrochemischen Sensors generiert. Fig. 3b zeigt die Messwerte der beiden Sensoren.

Der trockene Sensor zeigt ein deutlich verändertes Verhalten. Die Signalspitze (Peak) des Stroms ist reduziert. Die abfallende Flanke ist deutlich langsamer geworden. Es ist kein Unterschwinger mehr erkennbar. Auf Basis dieser veränderten Dynamik lässt sich ein langsamer Sensor erkennen.

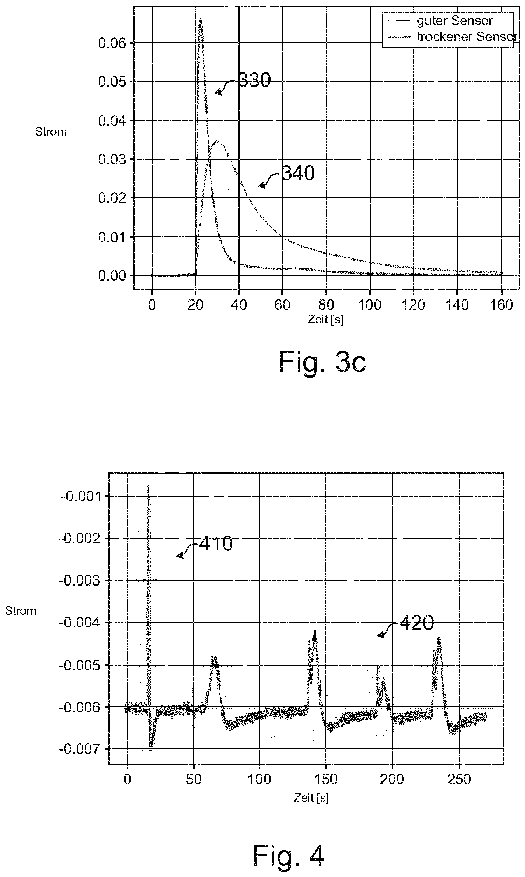

Im Anschluss an die Tests wurden die Sensoren noch einmal mit Trockengas mit ca. 380 µg/l Ethanol begast. Fig. 3c zeigt einen Vergleich der Ethanolkurve 330 des vitalen Sensors und der Ethanolkurve 340 des trockenen Sensors. Der trockene Sensor ist durch die Austrocknung deutlich langsamer geworden, ist aber dennoch noch messfähig.

In einem weiteren Versuch wurde ein Lötkolben als Heizquelle eingesetzt. Aufgrund dessen großer Temperatur, z.B. 400°C, strahlt der Lötkolben einen beachtlichen Teil seiner Energie als Wärmestrahlung ab. Der Lötkolben wurde statt LED berührungslos kurzfristig über den elektrochemischen Sensor gehalten. Der LED-Peak (Scheitelpunkt) unterscheidet sich dabei von dem Lötkolbenpeak. Der Lötkolbenpeak besteht aus zwei überlagerten Peaks. Diese waren im Versuchsaufbau weniger hoch als der LED-Peak, zudem fiel der LED-Peak schneller ab als die Lötkolben-Peaks. Fig. 4 zeigt die Messergebnisse des Versuchsaufbaus. Dabei ist Peak 410 der LED-Peak, und die nachfolgenden Peaks 420 sind Peaks, die von dem Lötkolben hervorgerufen wurden. Der Grund für die Signalform ist vermutlich, dass der erste schnelle Peak eine Folge von IR-Strahlung ist, welche in den Sensor eindringen und an der Elektrodenoberfläche zu einer Temperaturerhöhung führt. Der zweite Peak ist vermutlich eine Folge der Aufwärmung des Sensorgehäuses und der anschließenden langsamen Wärmeleitung. Dieser Effekt ist auch dann noch wirksam, wenn der Lötkolben schon längst wieder entfernt wurde. Im Gegensatz hierzu dringt der größte Teil der LED-Strahlung in den Sensor ein und reagiert direkt an der Elektrodenoberfläche, also im Sensorinneren. Dabei wird eine Thermospannung durch die Temperaturerhöhung induziert. Langsame Wärmeleitungseffekte treten nicht auf. Dies zeigt die Vorteile beim Einsatz einer LED als Strahlungsquelle.

In einem weiteren Versuch wurde das Gehäuse eines elektrochemischen Sensors im Wesentlichen lichtundurchlässig gemacht und dann mit einer LED bestrahlt. Bei einer Wiederholung des Versuchs konnte somit nur ein Bruchteil des LED-Lichts in den Sensor eindringen, stattdessen erwärmte sich das Gehäuse. Dabei war in den entsprechenden Messungen nur ein unwesentlicher Unterschied zwischen der Erwärmung über einen Lötkolben und der Erwärmung über die LED erkennbar. Dabei wurde die Peakhöhe des LED-Peaks deutlich reduziert, und die Gesamtdauer wurde erhöht.

Mehr Details und Aspekte des Konzepts werden in Verbindung mit dem Konzept oder Beispielen genannt, die vorher, z. B. Fig. 1 bis 2b, beschrieben wurden. Ausführungsbeispiele des Konzepts können ein oder mehrere zusätzliche optionale Merkmale umfassen, die ein oder mehreren Aspekten des vorgeschlagenen Konzepts oder der beschriebenen Beispiele entsprechen, wie sie vorher oder nachher beschrieben wurden.

Die Aspekte und Merkmale, die zusammen mit einem oder mehreren der vorher detaillierten Beispiele und Figuren beschrieben sind, können auch mit einem oder mehreren der anderen Beispiele kombiniert werden, um ein gleiches Merkmal des anderen Beispiels zu ersetzen oder um das Merkmal in das andere Beispiel zusätzlich einzuführen.

Beispiele können weiterhin ein Computerprogramm mit einem Programmcode zum Ausführen eines oder mehrerer der obigen Verfahren sein oder sich darauf beziehen, wenn das Computerprogramm auf einem Computer oder Prozessor ausgeführt wird. Schritte, Operationen oder Prozesse von verschiedenen, oben beschriebenen Verfahren können durch programmierte Computer oder Prozessoren ausgeführt werden. Beispiele können auch Programmspeichervorrichtungen, z. B. Digitaldatenspeichermedien, abdecken, die maschinen-, prozessor- oder computerlesbar sind und maschinenausführbare, prozessorausführbare oder computerausführbare Programme von Anweisungen codieren. Die Anweisungen führen einige oder alle der Schritte der oben beschriebenen Verfahren aus oder verursachen deren Ausführung. Die Programmspeichervorrichtungen können z. B. Digitalspeicher, magnetische Speichermedien wie beispielsweise Magnetplatten und Magnetbänder, Festplattenlaufwerke oder optisch lesbare Digitaldatenspeichermedien umfassen oder sein. Weitere Beispiele können auch Computer, Prozessoren oder Steuereinheiten, die zum Ausführen der Schritte der oben beschriebenen Verfahren programmiert sind, oder (feld-)programmierbare Logik-Arrays ((F)PLAs = (Field) Programmable Logic Arrays) oder (feld-)programmierbare Gate-Arrays ((F)PGA = (Field) Programmable Gate Arrays), die zum Ausführen der Schritte der oben beschriebenen Verfahren programmiert sind, abdecken.

Funktionen verschiedener in den Figuren gezeigter Elemente einschließlich jeder als "Mittel", "Mittel zum Bereitstellen eines Signals", "Mittel zum Erzeugen eines Signals", etc. bezeichneter Funktionsblöcke kann in Form dedizierter Hardware, z. B "eines Signalanbieters", "einer Signalverarbeitungseinheit", "eines Prozessors", "einer Steuerung" etc. sowie als Hardware fähig zum Ausführen von Software in Verbindung mit zugehöriger Software implementiert sein. Bei Bereitstellung durch einen Prozessor können die Funktionen durch einen einzelnen dedizierten Prozessor, durch einen einzelnen gemeinschaftlich verwendeten Prozessor oder durch eine Mehrzahl von individuellen Prozessoren bereitgestellt sein, von denen einige oder von denen alle gemeinschaftlich verwendet werden können. Allerdings ist der Begriff "Prozessor" oder "Steuerung" bei Weitem nicht auf ausschließlich zur Ausführung von Software fähige Hardware begrenzt, sondern kann Digitalsignalprozessor-Hardware (DSP-Hardware; DSP = Digital Signal Processor), Netzprozessor, anwendungsspezifische integrierte Schaltung (ASIC = Application Specific Integrated Circuit), feldprogrammierbare Logikanordnung (FPGA = Field Programmable Gate Array), Nurlesespeicher (ROM = Read Only Memory) zum Speichern von Software, Direktzugriffsspeicher (RAM = Random Access Memory) und nichtflüchtige Speichervorrichtung (storage) umfassen. Sonstige Hardware, herkömmliche und/oder kundenspezifische, kann auch eingeschlossen sein.

Ein Blockdiagramm kann zum Beispiel ein grobes Schaltdiagramm darstellen, das die Grundsätze der Offenbarung implementiert. Auf ähnliche Weise können ein Flussdiagramm, ein Ablaufdiagramm, ein Zustandsübergangsdiagramm, ein Pseudocode und dergleichen verschiedene Prozesse, Operationen oder Schritte repräsentieren, die zum Beispiel im Wesentlichen in computerlesbarem Medium dargestellt und so durch einen Computer oder Prozessor ausgeführt werden, ungeachtet dessen, ob ein solcher Computer oder Prozessor explizit gezeigt ist. In der Beschreibung oder in den Patentansprüchen offenbarte Verfahren können durch ein Bauelement implementiert werden, das ein Mittel zum Ausführen eines jeden der jeweiligen Schritte dieser Verfahren aufweist.

Es versteht sich, dass die Offenbarung mehrerer, in der Beschreibung oder den Ansprüchen offenbarter Schritte, Prozesse, Operationen oder Funktionen nicht als in der bestimmten Reihenfolge befindlich ausgelegt werden soll, sofern dies nicht explizit oder implizit anderweitig, z. B. aus technischen Gründen, angegeben ist. Daher werden diese durch die Offenbarung von mehreren Schritten oder Funktionen nicht auf eine bestimmte Reihenfolge begrenzt, es sei denn, dass diese Schritte oder Funktionen aus technischen Gründen nicht austauschbar sind. Ferner kann bei einigen Beispielen ein einzelner Schritt, Funktion, Prozess oder Operation mehrere Teilschritte, -funktionen, -prozesse oder -operationen einschließen und/oder in dieselben aufgebrochen werden. Solche Teilschritte können eingeschlossen sein und Teil der Offenbarung dieses Einzelschritts sein, sofern sie nicht explizit ausgeschlossen sind.

Weiterhin sind die folgenden Ansprüche hiermit in die detaillierte Beschreibung aufgenommen, wo jeder Anspruch als getrenntes Beispiel für sich stehen kann.

## Patentansprüche

1. Eine elektrochemische Sensoranordnung (10) für ein Atemalkoholmessgerät (100), die elektrochemische Sensoranordnung umfassend:
einen elektrochemischen Sensor mit zumindest zwei Elektroden (12; 14); und
eine Wärmequelle (16),
eine Steuervorrichtung (20),
wobei
die Wärmequelle derart angeordnet ist, dass sie, bei Aktivierung, selektiv eine der Elektroden (12) des elektrochemischen Sensors erwärmt,
**dadurch gekennzeichnet, dass**
die Steuervorrichtung ausgebildet ist die Wärmequelle zu aktivieren und eine Vitalität der Elektroden basierend auf der selektiven Erwärmung der einen Elektrode, nämlich basierend auf einer durch die selektive Erwärmung erzeugten Spannung zwischen den Elektroden und dem dadurch erzeugten Stromfluss, zu bestimmen.

2. Die elektrochemische Sensoranordnung gemäß Anspruch 1, wobei die Wärmequelle eine Licht-emittierende Diode, LED, ist.

3. Die elektrochemische Sensoranordnung gemäß Anspruch 1 oder 2, wobei die Steuervorrichtung ausgebildet ist, um das Verfahren gemäß einem der Ansprüche 6 bis 10 auszuführen.

4. Die elektrochemische Sensoranordnung gemäß einem der Ansprüche 1 bis 3, umfassend eine grafische Ausgabeeinheit (18), um eine Information über eine Vitalität des Sensors auszugeben, wobei die Information über die Vitalität der Elektroden basierend auf der selektiven Erwärmung der einen Elektrode bestimmt ist.

5. Ein Atemalkoholmessgerät umfassend die elektrochemische Sensoranordnung gemäß einem der Ansprüche 1 bis 4.

6. Ein Verfahren (200) zum Bestimmen einer Vitalität von Elektroden eines elektrochemischen Sensors innerhalb einer elektrochemischen Sensoranordnung gemäß einem der Ansprüche 1 bis 4, das Verfahren umfassend:
Aktivieren (210) der Wärmequelle über einen vordefinierten Zeitraum, um eine der Elektroden des Sensors selektiv zu erwärmen;
Bestimmen (220) eines Stromflusses zwischen den Elektroden des elektrochemischen Sensors, wobei der Stromfluss auf einer Spannung basiert, die von der einer selektiven Erwärmung der einen Elektrode durch die Wärmequelle hervorgerufen ist; und
Bestimmen (230) der Vitalität des Sensors basierend auf einer Signalform des Stromflusses.

7. Das Verfahren gemäß Anspruch 6, ferner umfassend Bereitstellen (240) einer Information über die Vitalität des Sensors über eine grafische Ausgabeeinheit.

8. Das Verfahren gemäß Anspruch 7, wobei die Information über die Vitalität des Sensors eine Information über eine geschätzte Restdauer einer Verwendbarkeit des elektrochemischen Sensors unter Berücksichtigung einer vorgegebenen minimalen Messgenauigkeit umfasst.

9. Das Verfahren gemäß einem der Ansprüche 6 bis 8, wobei das Verfahren durch ein Gerät ausgeführt wird, das den elektrochemischen Sensor und die Wärmequelle umfasst, wobei das Verfahren als Teil eines Selbst-Tests des Geräts ausgeführt wird.

10. Das Verfahren gemäß einem der Ansprüche 6 bis 9, ferner umfassend Bestimmen (250) eines Kompensationsparameters basierend auf der Vitalität des Sensors, wobei der Kompensationsparameter abbildet, inwiefern sich die Vitalität des Sensors auf die Messungen des Sensors auswirkt, wobei das Verfahren ferner ein Durchführen (255) von Messungen mit Hilfe des elektrochemischen Sensors unter Berücksichtigung des Kompensationsparameters umfasst.

## Claims

1. An electrochemical sensor assembly (10) for a respiratory alcohol measuring device (100), the electrochemical sensor assembly comprising:
an electrochemical sensor having at least two electrodes (12; 14); and a heat source (16),
a control device (20),
the heat source being arranged such that, upon activation, it selectively heats one of the electrodes (12) of the electrochemical sensor,
**characterized in that** the control device is designed to activate the heat source and to determine a vitality of the electrodes based on the selective heating of the one electrode, specifically based on a voltage between the electrodes generated by the selective heating and the current flow generated as a result.

2. The electrochemical sensor assembly according to claim 1, wherein the heat source is a light-emitting diode (LED).

3. The electrochemical sensor assembly according to claim 1 or claim 2, wherein the control device is designed to carry out the method according to any of claims 6 to 10.

4. The electrochemical sensor assembly according to any of claims 1 to 3, comprising a graphical output unit (18) for outputting information about a vitality of the sensor, wherein the information about the vitality of the electrodes is determined based on the selective heating of the one electrode.

5. A respiratory alcohol measuring device comprising the electrochemical sensor assembly according to any of claims 1 to 4.

6. A method (200) for determining a vitality of electrodes of an electrochemical sensor within an electrochemical sensor assembly according to any of claims 1 to 4,
the method comprising:
activating (210) the heat source over a predefined period of time in order to selectively heat one of the electrodes of the sensor;
determining (220) a current flow between the electrodes of the electrochemical sensor, wherein the current flow is based on a voltage caused by the selective heating of the one electrode by the heat source; and
determining (230) the vitality of the sensor based on a signal shape of the current flow.

7. The method according to claim 6, further comprising providing (240) information about the vitality of the sensor by means of a graphical output unit.

8. The method according to claim 7, wherein the information about the vitality of the sensor comprises information about an estimated remaining duration of usability of the electrochemical sensor taking into account a predefined minimum measurement accuracy.

9. The method according to any of claims 6 to 8, wherein the method is carried out by a device comprising the electrochemical sensor and the heat source, wherein the method is carried out as part of a self-test of the device.

10. The method according to any of claims 6 to 9, further comprising determining (250) a compensation parameter based on the vitality of the sensor, wherein the compensation parameter maps to what extent the vitality of the sensor affects the measurements of the sensor, wherein the method further comprises performing (255) measurements using the electrochemical sensor taking into account the compensation parameter.

## Revendications

1. Un ensemble capteur électrochimique (10) pour un éthylomètre (100), l'ensemble capteur électrochimique, comprenant :
un capteur électrochimique comportant au moins deux électrodes (12 ; 14) et une source de chaleur (16),
un dispositif de commande (20),
dans lequel la source de chaleur est disposée de telle sorte que, une fois activée, elle chauffe de manière sélective l'une des électrodes (12) du capteur électrochimique,
**caractérisé en ce que** le dispositif de commande est conçu pour activer la source de chaleur et pour déterminer une vitalité des électrodes sur la base du chauffage sélectif de l'une des électrodes, notamment sur la base d'une tension, générée par le chauffage sélectif, entre les électrodes et le flux de courant ainsi généré.

2. L'ensemble capteur électrochimique selon la revendication 1, dans lequel la source de chaleur est une diode électroluminescente, DEL.

3. L'ensemble capteur électrochimique selon la revendication 1 ou 2, dans lequel le dispositif de commande est conçu pour mettre en oeuvre le procédé selon l'une des revendications 6 à 10.

4. L'ensemble capteur électrochimique selon l'une des revendications 1 à 3, comprenant une unité de sortie graphique (18) pour sortir une information sur une vitalité du capteur, dans lequel l'information sur la vitalité des électrodes est déterminée sur la base du chauffage sélectif de l'une des électrodes.

5. Un éthylomètre comprenant l'ensemble capteur électrochimique selon l'une des revendications 1 à 4.

6. Un procédé (200) pour la détermination d'une vitalité d'électrodes d'un capteur électrochimique à l'intérieur d'un ensemble capteur électrochimique selon l'une des revendications 1 à 4,
le procédé comprenant :
l'activation (210) de la source de chaleur pendant une période prédéfinie pour chauffer de manière sélective l'une des électrodes du capteur ;
la détermination (220) d'un flux de courant entre les électrodes du capteur électrochimique, dans lequel le flux de courant est basé sur une tension qui est induite par celle d'un chauffage sélectif de l'une des électrodes par la source de chaleur ; et
la détermination (230) de la vitalité du capteur sur la base d'une forme d'onde du flux de courant.

7. Le procédé selon la revendication 6, comprenant en outre la fourniture (240) d'une information sur la vitalité du capteur sur une unité de sortie graphique.

8. Le procédé selon la revendication 7, dans lequel l'information sur la vitalité du capteur comprend une information sur une durée restante estimée d'une disponibilité du capteur électrochimique en tenant compte d'une précision de mesure minimale prédéterminée.

9. Le procédé selon l'une des revendications 6 à 8, dans lequel le procédé est mis en oeuvre par un appareil qui comprend le capteur électrochimique et la source de chaleur, dans lequel le procédé est mis en oeuvre dans le cadre d'un autotest de l'appareil.

10. Le procédé selon l'une des revendications 6 à 9, comprenant en outre la détermination (250) d'un paramètre de compensation sur la base de la vitalité du capteur, dans lequel le paramètre de compensation reproduit la manière dont la vitalité du capteur influe sur les mesures du capteur, dans lequel le procédé comprend en outre une réalisation (255) de mesures à l'aide du capteur électrochimique en tenant compte du paramètre de compensation.
